# EUROPEAN PATENT APPLICATION

(11) **EP 1 586 269 A1**
(43) Date of publication of application: **19.10.2005**
(21) Application number: 05252404.8
(22) Date of filing: 18.04.2005
(51) Int. Cl.: A61B 5/15

(54) **A cap for a dermal tissue lancing device**

(30) Priority: 16.04.2004 US 825899; 28.01.2005 US 45542; 28.01.2005 US 45544
(71) Applicant: LIFESCAN, INC., Milpitas, California 95035 (US)
(72) Inventor: Allen, John J., Minnesota 55118 (US); Menendez, Adolfo, Minnesota 55016 (US)
(74) Representative: Mercer, Christopher Paul

(57) **Abstract**

An apparatus for lancing a dermal tissue target site includes a dermal tissue lancing device with a housing, a lancet that is moveable with respect to the housing, and a cap. The cap has an opening therethrough for a portion of a lancet to pass through and a continuous saddle-contoured compression surface. The continuous saddle-contoured compression surface is configured for engaging a dermal tissue target site such that, when the cap contacts and is urged towards the dermal tissue target site, the continuous saddle-contoured compression surface applies substantially uniform pressure against the dermal tissue target site.

## Description

### CROSS-REFERENCE

This application claims the benefit of U.S. Application No. 11/045,542, filed January 28, 2005, which is incorporated herein by reference in its entirety and to which application we claim priority under 35 USC §120. This application claims the benefit of U.S. Application No. 11/045,544, filed January 28, 2005, which is incorporated herein by reference in its entirety and to which application we claim priority under 35 USC §120. This application is a continuation-in-part application of U.S. Application No. 10/825,899, filed April 16, 2004, which is incorporated herein by reference in its entirety and to which application we claim priority under 35 USC §120.

### BACKGROUND OF THE INVENTION

### 1. Field of the Invention

The present invention relates, in general, to lancing methods and, in particular, to methods for lancing dermal tissue.

### 2. Description of the Related Art

Conventional dermal tissue lancing methods generally employ lancing devices with a rigid housing and a lancet that can be armed and launched so as to protrude from one end of the lancing device. For example, conventional lancing devices can include a lancet that is mounted within a rigid housing such that the lancet is movable relative to the rigid housing along a longitudinal axis thereof. Typically, the lancet is spring loaded and launched, upon release of the spring, to penetrate (i.e., "lance") a target site (e.g., a dermal tissue target site). A biological fluid sample (e.g., a whole blood sample) can then be expressed from the penetrated target site for collection and analysis. Conventional lancing devices are described, for example, in U.S. Patent No. 5,730,753 to Morita, U.S. Patent No. 6,045,567 to Taylor et al. and U.S. Patent No. 6,071,250 to Douglas et al., each of which is incorporated fully herein by reference.

Lancing devices often include a cap with a distal end that engages the target site during use. Such a cap usually has an aperture (i.e., opening), through which the lancet protrudes during use. When a cap is engaged (i.e., contacted) with a target site, pressure is usually applied to the target site prior to launch of the lancet. This pressure urges the cap against the target site with the intent of creating a target site bulge within the opening of the cap. The lancet is then launched to penetrate the target site bulge. A biological fluid sample, typically blood, is thereafter expressed from the lanced target site bulge. The expressed biological fluid sample can then, for example, be tested for an analyte such as blood glucose.

However, conventional lancing methods employing such caps may not serve to reliably produce an adequate volume of biological fluid sample due to insufficient contact between the cap and the target site and/or non-uniform application of pressure on the target site by the cap. The design of conventional caps can also cause discomfort to a user during the lancing procedure. Furthermore, in order to obtain a sufficient volume of biological fluid sample, additional pressure (such as a pumping or milking action) usually must be applied either manually or mechanically to the target site following lancing. This additional pressure can serve to facilitate expression of an adequate volume of biological fluid sample. Examples of mechanical devices designed for such use are described in co-pending U.S. Application No. 10/653,023 (published as U.S. Patent Application Publication 2004/0249253 on December 9, 2004) and U.S. Patent 5,951,493, each of which is fully incorporated herein by reference. Unfortunately, such devices can be expensive to manufacture.

Still needed in the field, therefore, is a method for lancing a dermal tissue target site that enables a user to reliably obtain an adequate biological fluid sample (e.g., a whole blood sample) without subsequent manipulation of a target site. Furthermore, the method should be comfortable to the user.

### SUMMARY OF THE INVENTION

The present invention provides a cap for a dermal tissue lancing device, the dermal tissue lancing device including a housing and a lancet that is moveable with respect to the housing, the cap comprising:
a body with an opening therethrough for at least a portion of a lancet to pass through, the body having:
   a proximal end configured for engagement with the housing; and
   a distal end;
wherein the distal end includes:
a projection with a rim, the rim having a continuous saddle-contoured compression surface for engaging a dermal tissue target site, whereby, when the cap contacts and is urged towards the dermal tissue target site, the continuous saddle-contoured compression surface applies substantially uniform pressure against the dermal tissue target site.

Preferably the continuous saddle-countered compression surface is an elliptical continuous saddle-contoured compression surface.

Preferably the elliptical continuous saddle-contoured compression surface has a major axis and a minor axis and the ratio of the major axis to the minor axis is in the range of about 1.1 to 1.8.

Preferably the elliptical continuous saddle-contoured compression surface has a major axis and a minor axis and the major axis has a length in the range of about 10mm to 16mm and the minor axis has a length in the range of about 9mm to 13 mm.

Preferably the projection has a height in the range of 3mm to 5mm.

Preferably the continuous saddle-contoured compression surface has a saddle height in the range of from about 0.2mm to 0.8 mm.

Preferably the rim includes a lip extending into the opening.

Preferably the lip forms an angle alpha with a theoretical plane that is perpendicular to the opening, the angle alpha being the range of + 10 degrees to -10 degrees.

Preferably the cap is comprised of a rigid material selected from the group consisting of polystyrene materials, polycarbonate materials, polyester materials and combinations thereof.

Preferably the cap is comprised of a deformable material selected from the group consisting of elastomeric materials, polymeric materials, polyurethane materials, latex materials, silicone materials, and combinations thereof.

Caps, methods and apparatuses for lancing a dermal tissue target site according to embodiments of the present invention enable a user to reliably obtain an adequate volume of biological fluid sample (e.g., a whole blood sample) without subsequent manipulation of the dermal tissue target site (e.g., a dermal tissue target site on a user's finger). Furthermore, methods using the present invention are relatively comfortable to a user.

A method for lancing a dermal tissue target site according to an embodiment of the present invention includes providing a dermal tissue lancing device with a housing, a lancet that is moveable with respect to the housing, and a cap. In addition, the cap has an opening therethrough for at least a portion of a lancet to pass through and a continuous saddle-contoured compression surface.

The continuous saddle-contoured compression surface is configured for engaging a dermal tissue target site such that, when the cap contacts and is urged towards the dermal tissue target site, the continuous saddle-contoured compression surface applies substantially uniform pressure against the dermal tissue target site.

The method also includes contacting the cap with the dermal tissue target site such that the continuous saddle-contoured compression surface completely engages the target site, urging the cap towards the dermal tissue such that the continuous saddle-contoured compression surface applies essentially uniform pressure against the dermal tissue target site, thereby creating a target site bulge, and lancing the target site bulge.

The continuous saddle-contoured compression surface employed in the method has a three-dimensional profile that provides for reliable and complete contact between the cap and the dermal tissue target site and, hence, uniform application of pressure on the dermal tissue target site. The continuous saddle-contoured compression surface is particularly suited for contact with a dermal tissue target site of a user's finger. Since the continuous saddle-contoured compressions surface is complementary to the contour of a user's finger, the method relatively comfortable to a user.

The present invention also provides a method or apparatus for lancing a dermal tissue target site, the method comprising (or the apparatus comprising means for):
providing a dermal tissue lancing device, the dermal tissue lancing device including a housing, a lancet that is moveable with respect to the housing, and a cap, the cap having:
   an opening therethrough for at least a portion of a lancet to pass through; and
   a continuous saddle-contoured compression surface for engaging a dermal tissue target site such that, when the cap contacts and is urged towards the dermal tissue target site, the continuous saddle-contoured compression surface applies substantially uniform pressure against the dermal tissue target site;
   contacting the cap with a target site of the dermal tissue such that the continuous saddle-contoured compression surface completely engages the dermal tissue target site;
   urging the cap towards the dermal tissue target site such that the continuous saddle-contoured compression surface applies essentially uniform pressure against the dermal tissue target site, thereby creating a target site bulge; and
   lancing the target site bulge.

Preferably the providing step provides a dermal tissue lancing device with a continuous saddle-countered compression surface is an elliptical continuous saddle-contoured compression surface.

Preferably the providing step provides a dermal tissue lancing device with an elliptical continuous saddle-contoured compression surface that has a major axis and a minor axis and the ratio of the major axis to the minor axis is in the range of about 1.1 to 1.8.

Preferably the providing step provides a dermal tissue lancing device with an elliptical continuous saddle-contoured compression surface that has a major axis and a minor axis and the major axis has a length in the range of about 10mm to 16mm and the minor axis has a length in the range of about 9mm to 13 mm.

Preferably the providing step provides a dermal tissue lancing device wherein the projection has a height in the range of 3mm to 5mm.

Preferably the providing step provides a dermal tissue lancing device wherein the continuous saddle-contoured compression surface has a saddle height in the range of from about 0.2mm to 0.8 mm.

Preferably the providing step provides a dermal tissue lancing device with a rim that includes a lip extending into the opening.

Preferably the providing step provides a dermal tissue lancing device with a the lip that forms an angle alpha with a theoretical plane that is perpendicular to the opening, the angle alpha being the range of + 10 degrees to -10 degrees.

### BRIEF DESCRIPTION OF THE DRAWINGS

A better understanding of the features and advantages of the present invention will be obtained by reference to the following detailed description that sets forth illustrative embodiments, in which the principles of the invention are utilized, and the accompanying drawings, of which:
FIG. 1 is a simplified perspective view of a cap for use with a dermal tissue lancing device according to an embodiment of the present invention;
FIG. 2A is a top view of the cap illustrated in FIG 1;
FIG. 2B is a side view of the cap illustrated in FIG. 1 taken along line A-A of FIG. 2A;
FIG. 2C is a side view of the cap illustrated in FIG. 1 taken along line B-B of FIG. 2B;
FIG. 3 is a simplified perspective view of a cap for use with a dermal tissue lancing device according to another embodiment of the present invention;
FIG. 4A is a top view of the cap illustrated in FIG. 3;
FIG. 4B is a side view of the cap illustrated in FIG. 4A taken along line C-C of FIG. 4A;
FIG. 4C is a side view of the cap illustrated in FIG. 4A taken along line D-D of FIG. 4B; and
FIG. 5 is a flow diagram illustrating a sequence of steps in a process according to an embodiment of the present invention.

### DETAILED DESCRIPTION OF THE PREFERRED EMBODIMENTS

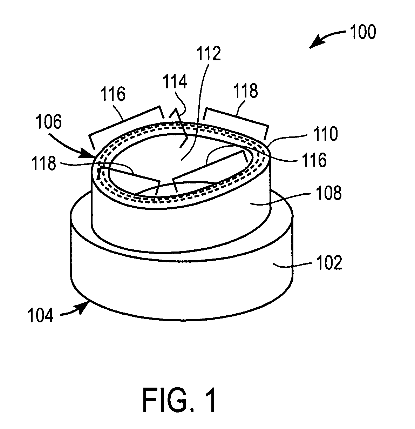

FIG. 1 is a simplified perspective view of a cap 100 for use with a dermal tissue lancing device (not shown) according to an exemplary embodiment of the present invention. Cap 100 includes a body 102 with a proximal end 104 and a distal end 106.

Cap 100 is configured to facilitate the flow of a biological fluid sample (e.g., a whole blood sample) out of a lanced dermal tissue target site with minimal or no manipulation (e.g., squeezing and/or milking) of the dermal tissue subsequent to lancing.

Proximal end 104 is configured to be removeably attached to an end of a dermal tissue lancing device (not shown) by, for example, slideably mounting, snap-fitting or screw-fitting proximal end 104 to the end of the dermal tissue lancing device. Alternatively, proximal end 104 of cap 100 can be configured for retention within a retainer (not shown) that is removeably attached to the end of a dermal tissue lancing device.

Once apprised of the present disclosure, one skilled in the art will recognize that a variety of conventional dermal tissue lancing devices can be readily modified for use with caps according to the embodiments of the present invention, including dermal tissue lancing devices described in the aforementioned U.S. Patent No's 5,730,753, 6,045,567 and 6,071,250. However, once apprised of the present invention, one skilled in the art will appreciate that the cap of the present invention is not limited to use with the lancing devices described therein. For example, embodiments of caps according to the present invention can be employed with lancing devices that include various techniques for expressing a biological fluid sample from a target site including, but not limited to, techniques that employ lancets, hollow needles, solid needles, micro-needles, ultrasonic extraction devices, or thermal extraction devices. Furthermore, caps according to embodiments of the present invention can be employed with a combined lancing device and integrated meter for testing an analyte (e.g., blood glucose). Such lancing devices are described in co-pending U.S. Application No. 10/825,899, which is hereby fully incorporated herein by reference.

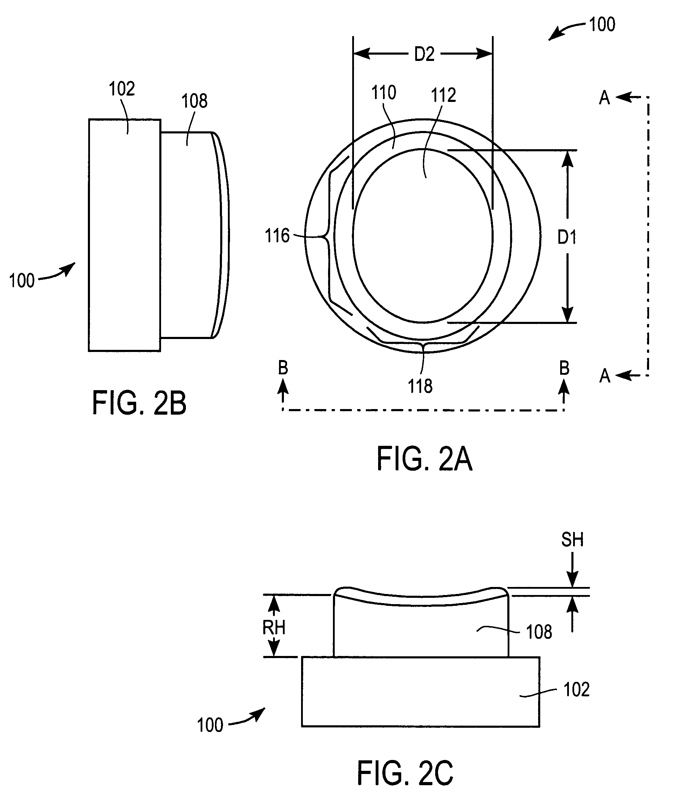

FIGs. 2A through 2C are simplified top and side views of cap 100. Distal end 106 is configured to engage with a dermal tissue target site (e.g., a dermal tissue target site on a user's finger) and includes a projection 108 with a rim 110 that defines an opening 112 for a lancet to pass through during lancing of the dermal tissue target site. For illustrative and explanation purposes only, opening 112 in the embodiment of FIGs. 1 through 2C is shown as elliptical or oval in shape, but can be any suitable shape. Rim 110 includes a continuous saddle-contoured compression surface 114 that forms a continuous ring for engaging a dermal tissue target site. Continuous saddle-contoured compression surface 114 accommodates the surface profile of a user's fingertip and, thus, improves the reliability and completeness of contact with the dermal tissue target site of a user's finger. The dashed lines of FIG. 1 indicate that continuous saddle-shaped compression surface 114 is a smooth curved surface.

Cap 100 can be formed of a relatively rigid material including, for example, polystyrene, polycarbonate, polyester or any combinations thereof. Cap 100 can also be formed of relatively resiliently deformable materials, including, but not limited to, elastomeric materials, polymeric materials, polyurethane materials, latex materials, silicone materials and combinations thereof. Cap 100 can be manufactured, for example, by injection molding, casting, machining and stereolithography techniques.

Referring to FIG. 2A, rim 110 is elliptical in shape with a major axis along line A-A and a minor axis along line B-B. Diameter D1 along the major axis is, therefore, larger than a diameter D2 along the minor axis. The dimensions of D 1 and D2 and their ratio are, for example, predetermined such that cap 100 conforms to the typical size of a user's finger. Moreover and in general, larger diameters (i.e., larger dimensions for D1 and D2) will result in a larger volume of biological fluid sample being expressed from a lanced target site. For an adult user's finger, diameter D1 is typically in the range of from about 10mm to 16mm and preferably in the range of from about 11mm to 12mm, while diameter D2 is typically in the range from about 9mm to 13mm and more typically in the range of from about 10mm to 11mm. The ratio of D1 to D2 is typically in the range of from about 1.1 to about 1.8.

Opposing first portions 116 of rim 110 are disposed on either side of the major axis and rise to a higher elevation (hereinafter referred to as saddle height SH) than opposing second portions 118 of rim 110 disposed on either side of the minor axis, as shown in FIG. 2C. Saddle height SH is predetermined such that cap 100 conforms, for example, to the curvature of a finger target site and such that pressure is uniformly distributed onto a target site (via continuous saddle-shaped compression surface 114 of rim 110) during use. For an adult's finger target site, saddle height SH typically ranges from about 0.2mm to about 0.8mm. The combination of an elliptically shaped rim and continuous saddle-contoured compression surface serve to provide reliable and complete contact between cap 100 and a target site on a user's finger and to provide for complete enclosure of a target site within opening 112.

Rim 110 is generally located at a height (hereinafter referred to as rim height RH) that is in the range of 3mm to 5mm above body 102. In other words, projection 108 of body 102 typically has a height in the range of 3mm to 5mm. Moreover thickness of rim 110 is, for example, typically in the range of 0.5mm to 3mm.

During use of cap 100, a dermal tissue target site of a user's finger (e.g., a fingertip target site) is placed along the major axis opposite opening 112. In other words, the longitudinal major axis of the user's finger is aligned with the major axis along line A-A of FIG. 2A. Cap 100 can also be placed on dermal tissue in other regions of the body including, for example, the forearm, abdomen or thigh. Although the saddle-shape of cap 100 is particularly beneficial for use with a finger target site, larger and more fleshy target sites (such as the forearm, abdomen and thigh) can readily conform to the saddle-shape of cap 100. Alternatively, D1, D 1 and SH can be predetermined such that cap 100 conforms to target sites on the forearm, abdomen or thigh.

When cap 100 is used in combination with a dermal tissue lancing device that includes means to control needle penetration depth during lancing, rim height RH can serve to provide sufficient separation between continuous saddle-contoured compression surface 114 and such a penetration depth control means, thereby ensuring adequate dermal tissue engagement during lancing. Nonlimiting examples of penetration depth control means and their use are described in US. Application No. 10/690,083, which is fully incorporated herein by reference. Rim height RH also provides the extension needed to adequately pressurize "fleshy" testing sites such as the forearm, abdomen or thigh.

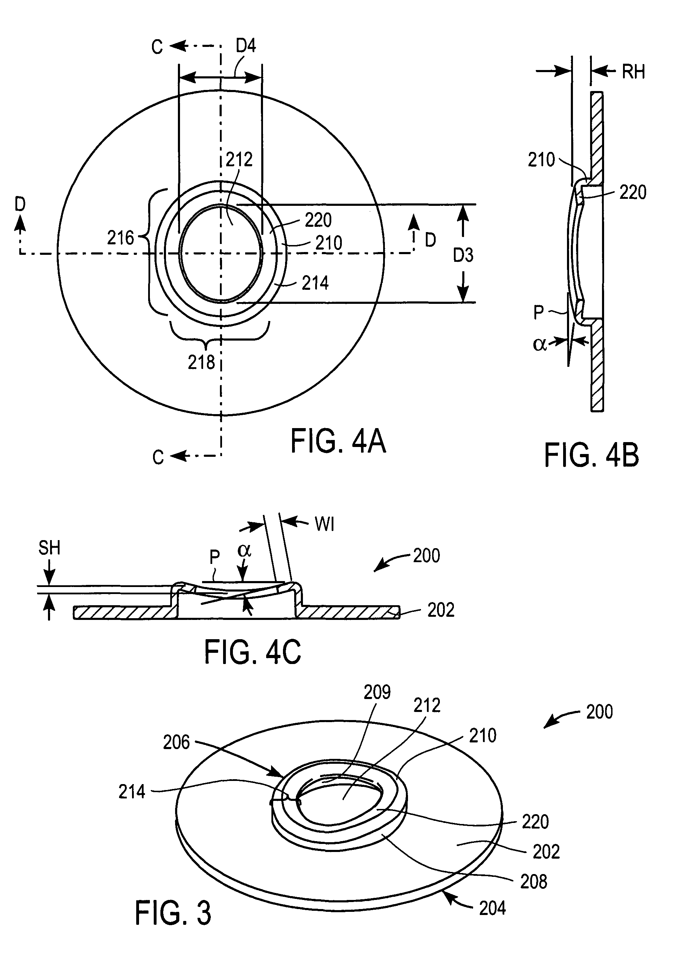

FIG. 3 depicts a cap 200 according to another exemplary embodiment of the present invention. FIGs. 4A, 4B and 4C are top and sides views of cap 200. Referring to FIGs. 3, and 4A through 4C, cap 200 includes a body 202 having a proximal end 204 and a distal end 206. Proximal end 204 is configured to be removeably or permanently attached to an end of a dermal tissue lancing device (not shown). Alternatively, proximal end 204 of cap 200 can be retained within a retainer (not shown) that is removeably attached to the end of the lancing device.

Distal end 206 is configured to engage with a dermal tissue target site and includes a substantially cylindrical projection 208 with a rim 210 that defines an opening 212 for the needle to pass through during lancing of the dermal tissue. Rim 210 includes a contoured compression surface 214 that forms a continuous ring for engaging a dermal tissue target site. Contoured compression surface 214 can accommodate the uneven surface of, for example, a fingertip and thus improve the reliability and completeness of contact with such an uneven dermal tissue target site surface.

Referring to FIG. 4A, a plane perpendicular to opening 212 includes a major axis along line C-C and a minor axis along line D-D. Diameter D3 of opening 212 along the major axis is larger than diameter D4 of opening 212 along the minor axis. Diameter D3 typically ranges from about 10mm to 16mm and usually ranges from about 11mm to 12mm. Diameter D4 typically ranges from about 9mm to 13mm and usually ranges from about 10mm to 11mm. The ratio of D3 to D4 is typically about 1.1 to 1.8.

Opposing first portions 216 of rim 210 disposed on either side of the major axis rise to a higher elevation (hereinafter referred to as saddle height SH) than opposing second portions 218 of rim 210 disposed on either side of the minor axis (see, for example, FIG. 4C). Saddle height SH typically ranges from about 0.2mm to about 0.8 mm.

Rim 210 has a height (hereinafter referred to as rim height RH) in the range of about 2mm to about 3mm above body 202. As with cap 100 described above, a target site of a user's finger is placed along the major axis opposite opening 212 during use of cap 200. However, cap 200 can also be placed on dermal tissue in other regions of the body including, for example, the forearm, abdomen or thigh.

When cap 200 is used in combination with a means to control needle penetration depth during lancing (not shown), rim height RH provides sufficient separation between contoured compression surface 214 and such needle penetration depth control means, ensuring adequate dermal tissue engagement during lancing. Examples of penetration depth control means and their use are further described in the aforementioned US. Application No. 10/690,083. Rim height RH can also provide the extension needed to adequately pressurize "fleshy" testing sites such as the forearm, abdomen or thigh.

Rim 210 further includes a lip 220 extending into opening 212. During use, lip 220 contacts a dermal tissue target site over a relatively small area and provides for a target site bulge to expand underneath of lip 220 within opening 212. It is postulated, without being bound, the area of contact between cap 100 and a target site may result in enhanced perfusion of a target site and, therefore, increased biological fluid expression from the target site. Lip 220 forms an angle α with a theoretical plane P that is perpendicular to opening 212 (see FIGs. 4B and 4C). Angle α can be in the range from -10 to +10 degrees such that lip 220 can extend below or above theoretical plane P and above or below opening 212. The width W1 of lip 220 (i.e., the distance lip 220 extends into opening 212) can range, for example, from about 0.2mm to about 2mm. Angle α and width W 1 are predetermined to simultaneously optimize the uniform application of pressure on a target site, allow for creation of a target site bulge within opening 212 and provide comfort to a user.

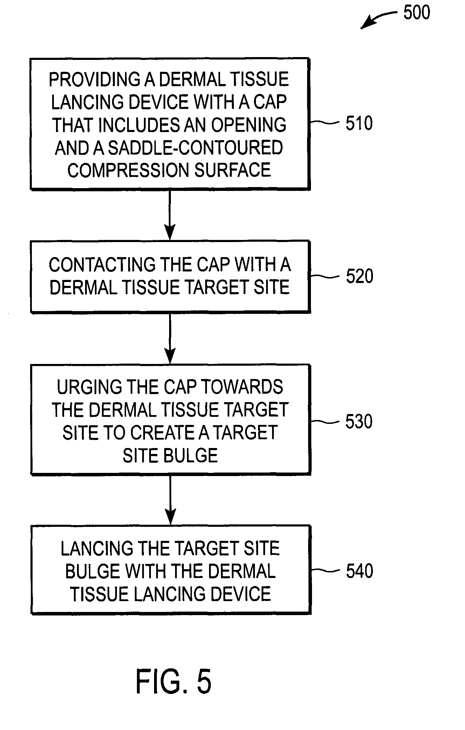

Referring to FIG. 5, a method 500 for lancing a dermal tissue target site (e.g., a dermal tissue target site on a user's finger) according to an exemplary embodiment of the present invention includes providing a dermal tissue lancing device that includes a cap with an opening therethrough and a continuous saddle-contoured compression surface as described above with respect to caps 100 and 200 (see step 510 of FIG. 5).

Next, as set forth in step 520, the cap of the dermal tissue lancing device is contacted with a dermal tissue target site such that the continuous saddle-contoured compression surface engages the dermal tissue target site in a substantially uniform manner.

Next, at step 530, the cap is urged towards the dermal tissue target site such that an essentially uniform pressure is applied to the dermal tissue target site creating a target site bulge. Further pressure on the cap pressurizes the bodily fluid trapped in the target site bulge. The pressure applied to the dermal tissue target site via the continuous saddle-contoured compression surface serves to trap dermal tissue inside the opening of the cap, thereby creating the target site bulge. Furthermore, the continuous saddle-contour shape of the compression surface and elliptical shape of the opening facilitate the reliable, uniform and complete engagement and application of pressure to the dermal tissue target site, thereby aiding in the subsequent expression of a biological fluid sample.

The target site bulge is then lanced with the lancing device (see step 540 of FIG, 5). Pressure applied to the target site via the continuous saddle-contoured compression surfaces facilitates expression of a bodily fluid sample from the lanced target site bulge.

Once apprised of the present disclosure, one skilled in the art will recognize that method 500 can be employ any suitable cap with a continuous saddle-contoured compression surface as described herein.

One embodiment of the present invention, a cap for a dermal tissue lancing device, the dermal tissue lancing device including a housing and a lancet that is moveable with respect to the housing, the cap comprising: a body with an opening therethrough for at least a portion of a lancet to pass through, the body having a proximal end configured for engagement with the housing; and a distal end; wherein the distal end includes: a projection with a rim, the rim having a continuous saddle-contoured compression surface for engaging a dermal tissue target site, whereby, when the cap contacts and is urged towards the dermal tissue target site, the continuous saddle-contoured compression surface applies substantially uniform pressure against the dermal tissue target site.

Another embodiment of the present invention, wherein the continuous saddle-countered compression surface is an elliptical continuous saddle-contoured compression surface.

Yet another embodiment of the present invention, wherein the elliptical continuous saddle-contoured compression surface has a major axis and a minor axis and the ratio of the major axis to the minor axis is in the range of about 1.1 to 1.8.

Yet a further embodiment of the present invention, wherein the elliptical continuous saddle-contoured compression surface has a major axis and a minor axis and the major axis has a length in the range of about 10mm to 16mm and the minor axis has a length in the range of about 9mm to 13 mm.

Yet a further embodiment of the present invention, wherein the projection has a height in the range of 3mm to 5mm.

Yet a further embodiment of the present invention, wherein the continuous saddle-contoured compression surface has a saddle height in the range of from about 0.2mm to 0.8 mm.

Yet a further embodiment of the present invention, wherein the rim includes a lip extending into the opening.

Yet a further embodiment of the present invention, wherein the lip forms an angle alpha with a theoretical plane that is perpendicular to the opening, the angle alpha being the range of + 10 degrees to -10 degrees.

Yet a further embodiment of the present invention, wherein the cap is comprised of a rigid material selected from the group consisting of polystyrene materials, polycarbonate materials, polyester materials and combinations thereof.

Yet a further embodiment of the present invention, wherein the cap is comprised of a deformable material selected from the group consisting of elastomeric materials, polymeric materials, polyurethane materials, latex materials, silicone materials, and combinations thereof.

It should be understood that various alternatives to the embodiments of the invention described herein may be employed in practicing the invention. It is intended that the following claims define the scope of the invention and that caps, methods and apparatuses within the scope of these claims and their equivalents be covered thereby.

## Claims

1. A cap for a dermal tissue lancing device, the dermal tissue lancing device including a housing and a lancet that is moveable with respect to the housing, the cap comprising:
a body with an opening therethrough for at least a portion of a lancet to pass through, the body having
a proximal end configured for engagement with the housing; and
a distal end;
wherein the distal end includes:
a projection with a rim, the rim having a continuous saddle-contoured compression surface for engaging a dermal tissue target site, whereby, when the cap contacts and is urged towards the dermal tissue target site, the continuous saddle-contoured compression surface applies substantially uniform pressure against the dermal tissue target site.

2. The cap of claim 1, wherein the continuous saddle-countered compression surface is an elliptical continuous saddle-contoured compression surface.

3. The cap of claim 2, wherein the elliptical continuous saddle-contoured compression surface has a major axis and a minor axis and the ratio of the major axis to the minor axis is in the range of about 1.1 to 1.8.

4. The cap of claim 2 or claim 3, wherein the elliptical continuous saddle-contoured compression surface has a major axis and a minor axis and the major axis has a length in the range of about 10mm to 16mm and the minor axis has a length in the range of about 9mm to 13 mm.

5. The cap of claim 2, claim 3 or claim 4, wherein the projection has a height in the range of 3mm to 5mm.

6. The cap of any one of the preceding claims, wherein the continuous saddle-contoured compression surface has a saddle height in the range of from about 0.2mm to 0.8 mm.

7. The cap of any one of the preceding claims, wherein the rim includes a lip extending into the opening.

8. The cap of claim 7, wherein the lip forms an angle alpha with a theoretical plane that is perpendicular to the opening, the angle alpha being the range of + 10 degrees to -10 degrees.

9. The cap of any one of the preceding claims, wherein the cap is comprised of a rigid material selected from the group consisting of polystyrene materials, polycarbonate materials, polyester materials and combinations thereof.

10. The cap of any one of the preceding claims, wherein the cap is comprised of a deformable material selected from the group consisting of elastomeric materials, polymeric materials, polyurethane materials, latex materials, silicone materials, and combinations thereof.
